# FASCICULE DE BREVET EUROPEEN

(11) **EP 0 542 855 B1**
(45) Date de publication et mention de la délivrance du brevet: **02.11.1994**
(21) Numéro de dépôt: 91914790.0
(22) Date de dépôt: 02.08.1991
(51) Int. Cl.: A61K 47/42

(54) **MICROCAPSULES A PAROI MIXTE D'ATELOCOLLAGENE ET DE POLYHOLOSIDES COAGULEE PAR UN CATION BIVALENT**
MIKROKAPSELN MIT WÄNDEN AUS MISCHUNGEN VON DURCH BIVALENTEN KATIONEN KOAGULIERTEN ATELOKOLLOGEN UND POLYHOLOSIDEN
MICROCAPSULES HAVING A COMBINED ATELOCOLLAGEN/POLYHOLOSIDE WALL COAGULATED BY A DIVALENT CATION

(30) Priorité: 03.08.1990 FR 9009997
(43) Date de publication de la demande: 26.05.1993
(73) Titulaire: COLETICA, 69007 Lyon (FR)
(72) Inventeur: ALLARD, Roland, F-69230 S.-Genis-Laval (FR); HUC, Alain, F-69110 Ste-Foy-des-Lyon (FR)
(74) Mandataire: Portal, Gérard
(86) Numéro de dépôt international: FR9100640
(87) Numéro de publication internationale: WO9202254

(56) Documents cités:
- EP-A- 0 209 726
- FR-A- 2 642 329
- GB-A- 1 388 580

## Description

La présente invention concerne essentiellement des microcapsules à paroi mixte d'atélocollagène et de polyholosides coagulée par un cation bivalent, un procédé de fabrication des microcapsules et des compositions cosmétiques ou pharmaceutiques ou alimentaires en contenant.

On sait que pour des applications pharmaceutiques et cosmétiques, de nombreux chercheurs travaillent à la mise au point d'encapsulation de substances actives. Parmi les effets recherchés pour une telle mise en oeuvre, on peut citer notamment l'amélioration de la biodisponibilité, la protection du principe actif dans une formule finie, la protection du principe actif dans l'organisme pour éviter notamment sa dégradation dans l'estomac, la libération différée, ou la libération lente pour effet prolongé.

Cette encapsulation peut être réalisée en incorporant ces principes actifs dans des microcapsules pour leur introduction dans des produits cosmétiques, des préparations pharmaceutiques ou des produits alimentaires destinés à diverses voies d'administration telles que la voie orale, la voie parentérale, l'application sur la peau et les muqueuses.

Dans l'état de la technique antérieure, on a déjà proposé diverses techniques pour la fabrication de microcapsules à l'aide de polymères synthétiques. Ces dernières substances permettent des fabrications industrielles aisées mais les microcapsules obtenues sont en général difficilement biodégradables et, lorsqu'elles le sont, elles donnent naissance à des produits de dégradation qui peuvent être toxiques, ou dont la toxicité n'est pas connue.

Ainsi, les travaux de recherche se sont orientés vers la réalisation de microcapsules à l'aide de substances naturelles biocompatibles et biodégradables. Dans ce cadre, les chercheurs ont utilisés des protéines. On peut se reporter par exemple au document FR-A-2 444 497 MARS ainsi que le document FR-A-2 527 438 CNRS.

Dans ces deux documents, la technique utilisée comporte trois étapes :
a) l'émulsification d'une solution aqueuse alcaline d'une protéine au sein d'un solvant organique non miscible à l'eau ;
b) la réticulation interfaciale des vésicules de l'émulsion grâce à un agent de réticulation qui est en général un dichlorure d'acide organique ; et enfin
c) l'isolation et le lavage des microcapsules obtenues grâce à des solvants appropriés.

Dans le premier document, la membrane est constituée uniquement de protéine, tandis que dans le second document elle est composée d'un mélange de protéines et de polyholosides.

Un autre procédé connu sous le nom de procédé EXTRAMET mis en oeuvre par la société EXTRAMET permet d'obtenir des microcapsules en coupant mécaniquement à l'aide d'un vibreur un écoulement laminaire réalisé par extrusion d'une solution de matériau polymérisable à travers une buse, ce qui provoque la formation de vésicules ou gouttelettes qui peuvent être ensuite rigidifiées par séchage ou par réticulation dans un bain contenant un agent réticulant dans lequel chutent les vésicules ou gouttelettes. Cette technique peut être appliquée à des polymères synthétiques ou à des protéines. L'encapsulation d'une substance active hydrosoluble dans une capsule protéique sera obtenue par dissolution dans la solution de protéines avant extrusion. Si la substance active est de forme huile ou bien si elle est en solution dans l'huile, elle sera encapsulée grâce à une coextrusion avec la solution protéique qui se trouve à l'extérieur du flux laminaire.

On observera que dans aucun des documents de l'état de la technique antérieure relatifs à la fabrication de microcapsules, et en particulier dans les deux documents précités FR-A-2 444 497 et FR-A-2 527 438, bien que les procédés soient appliqués à des protéines d'une manière générale, il n'est jamais cité ou fait allusion à l'emploi de collagène.

Les inventeurs de la présente invention ont essayé d'utiliser le collagène dans les procédés décrits dans les documents précédents ainsi qu'en utilisant la technique EXTRAMET.

Ces expériences se sont soldées par un échec car ces techniques ne sont pas applicables au collagène. En effet, pour obtenir une réticulation efficace, il est nécessaire de préparer des solutions de collagène dans un milieu fortement tamponné à des pH supérieurs ou égaux à 5,5.

Or, le collagène habituel, c'est-à-dire le collagène natif, précipite en partie et il est alors très difficile d'obtenir des mélanges homogènes. L'émulsion avec un liquide organique ne peut pas être réalisée, de sorte que les procédés décrits dans les documents précédents FR-A-2 444 497 et FR-A-2 527 438 ne peuvent être mis en oeuvre. Il en est également de même avec la technique d'extrusion laminaire EXTRAMET dans laquelle il est impossible d'obtenir un flux constant avec un mélange hétérogène.

Le déposant a également décrit dans le document FR-A-2 642 329 l'utilisation de solutions d'atélocollagène et de glycosaminoglycannes pour réaliser la fabrication des microcapsules par réticulation interfaciale à l'aide d'un agent de réticulation interfaciale habituellement constitué par un chlorure d'acide.

Malheureusement, cette technique de réticulation interfaciale nécessite l'emploi de chlorure de diacide pour la réalisation de capsules formées soit par dispersion, soit par extrusion. L'utilisation de chlorure de diacide nécessite l'emploi d'un agent organique support. Ces deux produits doivent être complètement éliminés par lavage, ce qui alourdit beaucoup le procédé industriel et en limite grandement son application.

La présente invention a donc pour but de résoudre le nouveau problème technique consistant en la fourniture d'une solution permettant de fabriquer des microcapsules dont la paroi comprend au moins en partie du collagène ou un produit de type collagénique présentant les mêmes propriétés que le collagène, selon un procédé de fabrication simplifié évitant l'emploi d'agent de réticulation tel que des chlorures d'acide qui doivent être éliminés.

La présente invention a encore pour but de résoudre le nouveau problème technique ci-dessus énoncé, avec l'utilisation de procédés de fabrication extrêmement simples, utilisables à l'échelle industrielle, et permettant en outre de régler à volonté la dimension des microcapsules, en particulier dans une plage de dimensions allant d'environ 100 à 3 000 µm, en particulier de 400 à 3 000 µm.

Selon la présente invention, on a découvert de manière totalement inattendue que les problèmes techniques énumérés ci-dessus pouvaient être résolus d'une manière extrêmement aisée, si l'on utilisait comme matière première une solution d'atélocollagène et de polyholosides, par exemple des glycosaminoglycannes, que l'on coagule à l'aide d'un cation bivalent, par exemple le calcium, le magnésium.

On obtient ainsi des microcapsules ayant des propriétés physicomécaniques remarquables, sans avoir à réaliser des procédures de lavage contrairement aux procédés antérieurement connus.

Ainsi, selon un premiet aspect, la présente invention a pour objet des microcapsules, caractérisées en ce qu'elles comprennent une paroi mixte d'atélocollagène et de polyholosides, par exemple des glycosaminoglycannes, coagulée par un agent de coagulation comprenant un cation bivalent.

La proportion de polyholosides, par exemple des glycosaminoglycannes, par rapport à l'atélocollagène peut varier de 15 à 50 % en poids.

Selon une variante de réalisation particulière, les polyholosides sont choisis parmi le groupe consistant en des glycosaminoglycannes tels que les glycosaminoglycannes de structure choisis parmi le groupe consistant du chondroïtine-4-sulfate, du chondroïtine-6-sulfate, du dermatane-sulfate, de l'héparane-sulfate, du kératane-sulfate ; ainsi que de l'héparine et de ses dérivés ; ou du dextrane.

Selon une variante de réalisation particulière, la paroi mixte est formée par coagulation d'un mélange atélocollagène-polyholosides, en particulier glycosaminoglycannes ou dextrane, en introduisant la solution de polyholosides dans la solution d'atélocollagène. En particulier, la concentration en polyholosides dans la solution de polyholosides est de 0,5 à 4 % en poids, encore mieux de 0,5 à 2 %, et encore de préférence est voisine de 1 %.

Selon une autre caractéristique particulière de l'invention, la solution d'atélocollagène initiale est une solution aqueuse d'atélocollagène ayant une concentration comprise entre 0,5 et 2 % en poids. Cette solution d'atélocollagène peut être obtenue selon l'invention par dissolution de fibres d'atélocollagène dans une solution aqueuse légèrement acide. Ces fibres d'atélocollagène sont par exemple mises en solution dans de l'acide acétique 0,1 M. Selon une variante de réalisation, l'atélocollagène peut être obtenu par digestion enzymatique de collagène.

Selon une autre variante de réalisation, on peut introduire dans la solution aqueuse d'atélocollagène et de glycosaminoglycannes initiale, ou à l'intérieur des microcapsules, un ou plusieurs principes actifs souhaités à l'état de solution, de suspension ou d'émulsion, en particulier une ou plusieurs substances d'intérêt cosmétique, pharmaceutique ou alimentaire.

Selon un autre mode de réalisation particulier de l'invention, le cation bivalent précité utilisé pour coaguler la solution d'atélocollagène-polyholosides est un cation bivalent choisi parmi le groupe consistant de calcium, magnésium, manganèse, baryum, zinc, en particulier sous forme de sel tel que chlorure ou sulfate. Des sels préférés sont les sels de chlorure. L'agent coagulant est de préférence présent dans un bain de coagulation à une concentration comprise entre 0,1 et 1 % en poids. Plus cette concentration sera élevée plus les capsules obtenues seront dures et plus leur résistance mécanique sera importante.

Ces microcapsules peuvent également comprendre une troisième substance coagulable par un agent coagulant, de préférence un cation bivalent, en particulier un alginate de sodium ou de la caséine.

Une composition initiale homogène d'atélocollagène, et de polyholosides comprendra alors les constituants suivants destinés à former la paroi des microcapsules :

| | |
|---|---|
| - atélocollagène | 1 à 1,5 % en poids |
| - polyholosides | 0,3 à 0,5 % en poids |
| - substance coagulable | 0,2 à 0,4 % en poids |

La présente invention concerne également un procédé de fabrication de microcapsules, caractérisé en ce qu'il comprend les étapes successives suivantes :
a) on prépare séparément une solution d'atélocollagène, et une solution de polyholosides ;
b) on mélange la solution d'atélocollagène avec la solution de polyholosides de manière à former une solution homogène d'atélocollagène et de polyholosides ;
c) on prépare un bain de coagulation contenant un agent de coagulation comprenant un cation bivalent ;
d) on forme des gouttelettes individuelles à partir de la solution homogène d'atélocollagène et de polyholosides que l'on fait tomber dans ledit bain de coagulation en obtenant ainsi des microcapsules par coagulation de l'atélocollagène et des polyholosides sous l'effet de l'agent de coagulation ; et
e) on sépare les microcapsules par tout moyen approprié, notamment par décantation naturelle après avoir éventuellement effectué un ou plusieurs lavages.

Selon une caractéristique avantageuse du procédé selon l'invention, l'agent coagulant est un cation bivalent de préférence choisi parmi le groupe consistant de calcium, de magnésium, de manganèse, de baryum, de zinc, en particulier sous forme de sel tel qu'un sel de chlorure ou de sulfate, le sel de chlorure étant préféré. La concentration du cation bivalent dans le bain de coagulation est avantageusement comprise entre 0,1 et 1 % en poids.

Selon un mode de réalisation particulier de l'invention, on mélange également dans la solution d'atélocollagène et de polyholosides, une substance coagulable par l'agent de coagulation, en particulier un alginate de sodium ou de la caséine.

Selon un autre mode de réalisation particulièrement avantageux du procédé selon l'invention, on forme les gouttelettes individuelles précitées par une extrusion laminaire de la solution homogène d'atélocollagène et de polyholosides à travers une buse d'extrusion tout en soumettant l'écoulement laminaire à des vibrations pour disloquer l'écoulement laminaire en lesdites gouttelettes individuelles.

Par ailleurs, selon une autre variante de réalisation permettant d'encapsuler un principe actif, on réalise à travers une buse d'extrusion une coextrusion laminaire de la solution homogène d'atélocollagène et de polyholosides et de la substance à encapsuler, tout en soumettant l'écoulement laminaire à des vibrations pour disloquer l'écoulement laminaire en gouttelettes individuelles.

Selon une autre variante de réalisation des procédés selon l'invention, on réalise le mélange atélocollagène-polyholosides en introduisant la solution de polyholosides dans la solution d'atélocollagène.

Selon un mode de réalisation particulier, la solution de polyholosides est préparée par mise en solution du polyholoside, de de préférence obtenu à l'état sec, par exemple en ayant été lyophilisé, dans une solution aqueuse dont le pH est réglé de sorte qu'après mélange avec la solution d'atélocollagène, le pH du mélange soit compris entre 5,5 et 10. De préférence, la solution aqueuse est une solution tampon basique. Cette solution tampon basique peut être une solution aqueuse de soude ou de préférence une solution aqueuse d'un tampon basique obtenu par neutralisation d'un acide faible avec une base forte, comme par exemple le carbonate de sodium, l'acétate de sodium ou le citrate de sodium, ou dans des solutions de phosphates de sodium et de potassium.

Selon une autre caractéristique avantageuse des procédés selon l'invention, la concentration en polyholosides par rapport à la concentration en atélocollagène est de 15 à 50 % en poids.

Selon une autre caractéristique avantageuse des procédés selon l'invention, la concentration en polyholosides dans la solution de polyholosides est de 0,5 à 4 %, encore mieux de 0,5 à 2 %, et encore de préférence est voisine de 1 %.

Selon une autre caractéristique des procédés de l'invention, la solution d'atélocollagène est une solution aqueuse d'atélocollagène ayant une concentration comprise entre 0,5 et 2 % en poids. Cette solution d'atélocollagène peut être obtenue selon l'invention par dissolution de fibres d'atélocollagène dans une solution aqueuse légèrement acide.

Selon un mode de réalisation particulier, ces fibres d'atélocollagène sont mises en solution dans de l'acide acétique 0,1 M.

Selon un autre mode de réalisation particulier des procédés selon l'invention, l'atélocollagène est obtenu par digestion enzymatique de collagène.

Selon une variante de réalisation particulière, les polyholosides utilisés selon l'invention sont choisis parmi les glycosaminoglycannes de structure choisis parmi le groupe consistant du chondroïtine-4-sulfate, du chondroïtine-6-sulfate, du dermatane-sulfate, de l'héparane-sulfate, du kératane-sulfate ; ainsi que de l'héparine et ses dérivés ; ou le dextrane.

On peut introduire dans la solution aqueuse d'atélocollagène et de polyholosides un ou plusieurs principes actifs souhaités à l'état de solution, de suspension ou d'émulsion, en particulier une ou plusieurs substances d'intérêt cosmétique, pharmaceutique ou alimentaire.

En particulier, dans la technique d'extrusion précitée, on peut réaliser une extrusion de la substance à encapsuler incorporée à l'intérieur du flux laminaire d'atélocollagène et de polyholosides destinés à constituer la paroi des microcapsules.

Dans le cas où la phase huileuse est la phase encapsulée, il est possible d'incorporer une ou plusieurs substances d'intérêt cosmétique, pharmaceutique ou alimentaire à l'état de solution de suspension ou d'émulsion dans cette phase huileuse.

En particulier, dans la technique d'extrusion précitée, on peut réaliser une coextrusion de la substance active dissoute, en suspension ou sous forme émulsionnée dans la phase huileuse, à l'intérieur du flux laminaire d'atélocollagène et de polyholosides destinés à constituer la paroi des microcapsules.

Enfin, selon un troisième aspect, la présente invention concerne encore une composition cosmétique ou une composition pharmaceutique, caractérisée en ce qu'elle comprend des microcapsules à paroi mixte atélocollagène-polyholosides coagulée par un agent coagulant comprenant un cation bivalent. De préférence, ces microcapsules contiennent au moins en partie un principe actif, en particulier un principe actif cosmétique , un principe actif pharmaceutique, ou alimentaire.

D'autres buts, caractéristiques et avantages de l'invention apparaîtront clairement à la lumière de la description explicative qui va suivre faite en référence à plusieurs exemples de réalisation de l'invention donnés simplement à titre d'illustration et qui ne sauraient donc en aucune façon limiter la portée de l'invention. Dans les exemples, tous, les pourcentages sont donnés en poids, sauf indication contraire.

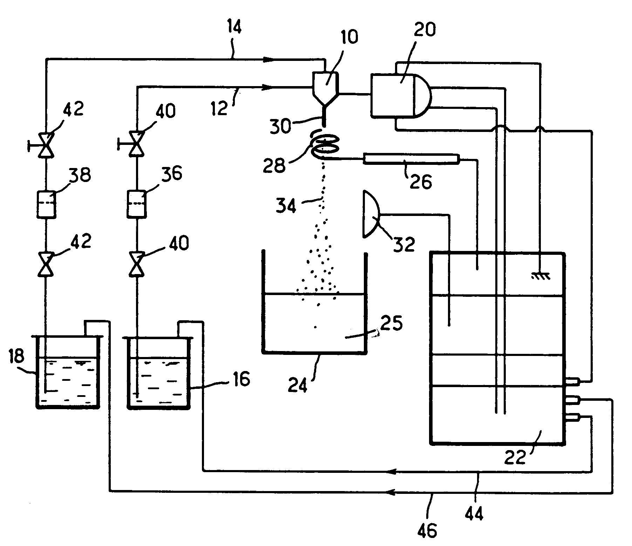

Les exemples de réalisation seront décrits en relation avec la figure unique annexée représentant schématiquement un appareil de fabrication de microcapsules selon la technique d'extrusion d'un écoulement laminaire type EXTRAMET.

### Exemple 1 selon l'invention

Dans cet exemple, on fabrique des microcapsules ayant un diamètre moyen de 600 µm renfermant de l'huile de bourrache.

### a) Préparation du collagène déréticulé ou atélocollagène

La peau de veau fraîchement abattu est soumise à épilation chimique dans un bain contenant 3 % de sulfure de sodium et 4 % de chaux, la proportion étant de 100 g de peau pour 200 cm³ de solution. Le derme est alors isolé du reste de la peau par une opération de refendage grâce à une scie tournante à ruban.

Le tissu obtenu est broyé et extrudé à travers une grille comportant des trous de 4 mm. Le broyat est alors mis en contact pendant 3 semaines avec un lait de chaux saturé à raison de 1 kg pour 4 l de solution. La peau ainsi traitée est séparée du surnageant par une centrifugation en continu sous une accélération de 2 000 g à l'aide d'une décanteuse tournant à 4 000 tour/min. Le culot est ensuite soumis à deux lavages à l'eau courante dans une cuve inox sous agitation lente à raison de 1 kg pour 4 l de bain. Le broyat est alors soumis à deux traitements du tampon phosphate pH 7,8 (21,7 g/l de Na₂HPO₄ et 0,78 g/l de KH₂PO₄) dans les mêmes conditions que pour le lavage à l'eau. Le culot est alors lavé par deux bains d'eau désionisée et stérile. Le broyat obtenu est placé dans une solution d'acide acétique (0,5 g/l, pH 3,4) à raison de 1 kg pour 20 l de bain. Après 5 minutes d'agitation, le surnageant est séparé du culot par décantation en continu selon la technique précédente. Le collagène est alors précipité par le surnageant par addition de chlorure de sodium sec dans une proportion de 10 % environ par rapport au bain. Après décantation par gravité, les fibres obtenues sont dialysées contre de l'eau désionisée et stérile à l'aide de membranes de dialyse, de préférence formées par des boyaux dont le seuil de coupure est compris entre 6 000 et 8 000 daltons.

### b) Préparation du chondroïtine-4-sulfate

Des cloisons nasales d'agneau débarrassées des tissus musculaires et adipeux sont hachées et broyées par extrusion à travers une grille comportant des trous de 4 mm ; le broyat est placé pendant 24 heures à une température de 6^{o}C dans un tampon de chlorure de potassium (11,8 g/l de KCL, 78,8 mg/l de cystéine, ETDA 180 mg/l) renfermant 1 % de papaïne "MERCK". La proportion étant de 130 g de broyat pour 1 l de tampon.

Le surnageant est séparé du culot par centrifugation en continu à l'aide d'une décanteuse tournant à 4 000 tour/min. Au surnageant sont alors ajoutés 40 g/l d'acide trichloracétique. Le précipité est éliminé par centrifugation en continu selon la technique précédente. Le surnageant est neutralisé à l'aide de soude en pastille. Le mélange est alors dialysé contre de l'eau désionisée et stérile à l'aide de boyaux dont le seuil de coupure est compris entre 6 000 et 8 000 daltons. La solution dialysée est lyophilisée. Le chondroïtine-4-sulfate est obtenu à l'état sec.

### c) Préparation de la solution homogène d'atélocollagène et de chondroïtine-4-sulfate en milieu tamponné à pH 7,5

L'atélocollagène sous forme de fibre provenant des boyaux de dialyse est mis en solution dans une solution aqueuse d'acide acétique 0,1 M de manière à obtenir une concentration d'atélocollagène de 3,2 %.

Cette solution est diluée par une solution de chondroïtine-4-sulfate dans la soude dont le volume et les concentrations sont tels que les concentrations finales du mélange homogène d'atélocollagène et de chondroïtine-4-sulfate soient celles données dans le tableau ci-dessous et que le pH du milieu soit voisin de 7,5 :

| | |
|---|---|
| - atélocollagène | 1,5 % |
| - chrondroïtine-4-sulfate | 0,5 % |
| - alginate de sodium | 0,3 % |
| - Nippagin® | 0,4 % |
| - eau désionisée | le solde. |

Le pH de la solution est ajusté à 7,5 soit par la soude, soit par l'acide chlorhydrique. On prépare ainsi 2 kg de cette solution.

### d) Préparation de l'agent coagulant

On dissout 55,5 g de chlorure de calcium ainsi que 10 g d'alcool éthylique dans 5 l d'eau désionisée. A la surface est déposée une couche de 1 à 2 cm d'épaisseur d'un ester d'acide gras disponible dans le commerce, par exemple connu sous la dénomination commerciale DRAGOXAT® vendu par la société DRAGOCO. On prépare également un deuxième bain identique.

### e) Coextrusion et coagulation des capsules

Pour ce faire, on utilise l'appareil Extramet schématisé à la figure unique annexée.

Cet appareil comprend essentiellement une buse d'extrusion 10 permettant de réaliser une coextrusion par la présence de deux orifices concentriques alimentés séparément par deux conduits d'alimentation 12, 14 servant par exemple respectivement à l'alimentation externe en solution d'atélocollagène-glycosaminoglycannes selon l'invention depuis un réservoir 16, et à l'intérieur un principe actif par exemple de l'huile de bourrache depuis un réservoir de principe actif 18. A cette buse 10, est associé un dispositif vibreur 20 commandé par des moyens de commande 22. Cet appareil comprend également un bain réticulant 24 disposé à distance sous la buse 10 dans lequel est disposée la solution d'agent coagulant 25.

Cet appareil comprend également une électrode 26 à extrémité 28 hélicoïdale disposée concentriquement à l'écoulement du flux laminaire 30 coextrudé de la buse 10 de manière à séparer les gouttelettes générées par le vibreur 20. On peut également prévoir un dispositif de stroboscopie par flash 32 pour observer visuellement les gouttelettes ainsi générées tombant dans le bain de coagulation 25.

Le débit de la solution homogène d'atélocollagène et du glycosaminoglycanne présents dans le réservoir 16 est de 2,4 l/h et celui de l'huile de bourrache présent dans le réservoir 18, constituant le principe actif, est de 1,2 l/h. La fréquence de vibration du fibreur 20 est de 230 MHz. Les diamètres des deux orifices concentriques sont de 400 et 600 µm.

Les gouttelettes 34 générées par le vibreur 20 à partir de l'écoulement laminaire réalisé dans la buse de coextrusion 10 sont reçues dans 1 l de bain de coagulation 25 maintenu sous agitation. Le bain est renouvelé après extrusion de 1 kg de la solution homogène d'atélocollagène et de glycosaminoglycannes.

### f) Lavage et stockage

Les capsules récupérées par filtration sont placées dans un bain de 10 l d'eau désionisée et maintenues sous agitation pendant 15 min. Au bout de ce laps de temps, elles sont à nouveau récupérées par filtration et placées dans un bain de 2 l d'eau désionisée dans lequel a été préalablement dissous 1 % d'une solution de phénonip® dans du propylèneglycol. Les volumes de ces deux derniers composés étant égaux. Dans de telles conditions, les capsules peuvent être stockées à température ambiante.

### Exemple 2 selon l'invention

Fabrication de microcapsules de diamètre moyen 400 µm renfermant un extrait de Ginko Biloba (Sté Alban Müller).

### a) Préparation de la solution homogène d'atélocollagène et de chondroïtine-4-sulfate en milieu tamponné à ph 9,8

1 kg de cette solution homogène est préparé comme décrit à l'exemple 1. Dans cette solution sont dissous 7 % de l'extrait de Ginko Biloba et le pH est à nouveau ajusté à 7,5.

### b) Extrusion et coagulation des microcapsules

On réalise l'extrusion et la coagulation des microcapsules dans les mêmes conditions que celles décrites à l'exemple 1, sauf en ce qui concerne le débit qui est de 2 l/h pour la solution homogène d'atélocollagène et de chondroïtine-4-sulfate contenant l'extrait de Ginko Biloba.

### c) Lavage et stockage des microcapsules

Les capsules sont reçues dans un bain de coagulation de 5 l identique à celui décrit dans l'exemple 1. Elles sont lavées et stockées dans les mêmes conditions.

Pour une utilisation dans des compositions pharmaceutiques, ces microcapsules permettent lors d'un emploi par voie orale de réaliser un masquage du goût du principe actif, ainsi qu'une protection dans l'estomac ou un effet différé grâce à une gastrorésistance qui peut être obtenue par une coagulation appropriée.

On peut également réaliser des compositions destinées à diverses voies d'administration telles que la voie orale, la voie parentérale, l'application sur la peau et les muqueuses.

La présente invention concerne encore d'une manière générale un procédé de préparation d'une composition cosmétique, pharmaceutique, ou alimentaire, caractérisé en ce qu'on incorpore au moins en partie des microcaspsules à paroi mixte atélocollagène-polyholosides coagulés dans lesquelles on a de préférence encapsulé au moins en partie une substance présentant un intérêt cosmétique, pharmaceutique ou alimentaire.

Ces microcapsules permettent également la protection de substances fragiles telles que les huiles essentielles susceptibles d'entrer dans la composition des aliments.

D'autres utilisations de ces microcapsules apparaîtront clairement à l'homme de l'art.

## Revendications

1. Microcapsules, caractérisées en ce qu'elles comprennent une paroi mixte d'atélocollagène et de polyholosides, en particulier des glycosaminoglycannes, coagulée par un agent de coagulation comprenant un cation bivalent.

2. Microcapsules selon la revendication 1, caractérisées en ce que le cation bivalent précité est choisi parmi le groupe consistant de calcium, de magnésium, de manganèse, de baryum, de zinc, en particulier sous forme d'un sel tel que sel de chlorure ou de sulfate.

3. Microcapsules selon la revendication 1 ou 2, caractérisées en ce que la proportion de polyholosides par rapport à l'atélocollagène peut varier de 15 à 50 % en poids.

4. Microcapsules selon l'une des revendications 1 à 3, caractérisées en ce que les polyholosides précités sont choisis parmi des glycosaminoglycannes et du dextrane, les glycosaminoglycannes sont de préférence choisis parmi les glycosaminoglycannes de structure choisis parmi le groupe consistant du chondroïtine-4-sulfate, du chondroïtine-6-sulfate, du dermatane-sulfate, de l'héparane-sulfate, du kératane-sulfate ; ainsi que de l'héparine et ses dérivés.

5. Microcapsules selon l'une des revendications 1 à 4, caractérisées en ce que la paroi comprend en outre une substance coagulable par l'agent de coagulation, en particulier choisie parmi un alginate de sodium ou de la caséine.

6. Microcapsules selon l'une des revendications 1 à 5, caractérisées en ce qu'un ou plusieurs principes actifs à l'état de solution, de suspension ou d'émulsion, en particulier une ou plusieurs substances d'intérêt cosmétique, pharmaceutique ou alimentaire, est présent dans la paroi mixte ou à l'intérieur des microcapsules.

7. Microcapsules selon la revendication 5 ou 6, caractérisées en ce que les constituants de la paroi des microcapsules sont les suivants :
| | |
|---|---|
| - atélocollagène | 1 à 1,5 % en poids |
| - polyholosides | 0,3 à 0,5 % en poids |
| - substance coagulable | 0,2 à 0,4 % en poids. |

8. Procédé de fabrication de microcapsules, caractérisé en ce qu'il comprend les étapes successives suivantes :
a) on prépare séparément une solution d'atélocollagène, et une solution de polyholosides ;
b) on mélange la solution d'atélocollagène avec la solution de polynolosides, de manière à former une solution homogène d'atélocollagène et de polyholosides ;
c) on prépare un bain de coagulation contenant un agent de coagulation comprenant un cation bivalent ;
d) on forme des gouttelettes individuelles à partir de la solution homogène d'atélocollagène et de polyholosides que l'on fait tomber dans ledit pain de coagulation en obtenant ainsi des microcapsules par coagulation de l'atélocollagène et des polyholosides sous l'effet de l'agent de coagulation ; et
e) on sépare les microcapsules par tout moyen approprié, notamment par décantation naturelle après avoir éventuellement effectué un ou plusieurs lavages.

9. Procédé selon la revendication 8, caractérisé en ce qu'on forme les gouttelettes individuelles par extrusion laminaire de la solution homogène d'atélocollagène et de polyholosides à travers une buse d'extrusion tout en soumettant l' écoulement laminaire à des vibrations pour disloquer l'écoulement laminaire en gouttelettes individuelles.

10. Procédé selon la revendication 8 ou 9, caractérisé en ce qu'on forme des gouttelettes individuelles encapsulant une substance en réalisant à travers une buse d'extrusion une coextrusion laminaire de la solution homogène d'atélocollagène et de polyholosides et de la substance encapsulée, tout en soumettant l'écoulement laminaire à des vibrations pour disloquer l'écoulement laminaire en lesdites gouttelettes individuelles encapsulant ladite substance.

11. Procédé selon l'une des revendications 8 à 10, caractérisé en ce qu'on réalise le mélange atélocollagène-polyholosides en introduisant la solution de polyholosides dans la solution d'atélocollagène.

12. Procédé selon l'une des revendications 8 à 11, caractérisé en ce que la solution de polyholosides est préparée par mise en solution du polyholosides, de préférence obtenu à l'état sec, par exemple en ayant été lyophilisé, dans une solution aqueuse dont le pH est réglé de sorte qu'après mélange avec la solution d'atélocollagène, le pH du mélange soit compris entre 5,5 et 10 ; cette solution aqueuse est de préférence une solution tampon basique.

13. Procédé selon la revendication 12, caractérisé en ce que la solution tampon précitée est choisie parmi une solution aqueuse de soude ou une solution aqueuse d'un tampon obtenue par neutralisation d'un acide faible avec une base forte, comme par exemple le carbonate de sodium, l'acétate de sodium ou le citrate de sodium, les phosphates de sodium et de potassium.

14. Procédé selon l'une des revendications 11 à 13, caractérisé en ce que la concentration en polyholosides par rapport à la concentration en atélocollagène est de 15 à 50 % en poids.

15. Procédé selon l'une des revendications 10 à 14, caractérisé en ce que la concentration en polyholosides dans la solution de polyholosides est de 0,5 à 4 %, encore mieux de 0,5 à 2 %, et encore de préférence est voisine de 1 %.

16. Procédé selon l'une des revendications 10 à 15, caractérisé en ce que la solution d'atélocollagène est une solution aqueuse d'atélocollagène ayant une concentration comprise entre 0,5 et 2 % en poids.

17. Procédé selon la revendication 16, caractérisé en ce que la solution d'atélocollagène est obtenue par dissolution de fibres d'atélocollagène dans une solution aqueuse légèrement acide, telle que de l'acide acétique 0,1 M.

18. Procédé selon l'une des revendications 8 à 17, caractérisé en ce que les polyholosides sont choisis parmi les polyholosides de structure choisis parmi le groupe consistant du chondroïtine-4-sulfate, du chondroïtine-6-sulfate, du dermatane-sulfate, de l'héparane-sulfate, du kératane-sulfate ; ainsi que de l'héparine et ses dérivés ; ou le dextrane.

19. Procédé selon l'une des revendications 8 à 18, caractérisé en ce qu'on introduit dans la solution aqueuse d'atélocollagène et de polyholosides un ou plusieurs principes actifs souhaités à l'état de solution, de suspension ou d'émulsion, en particulier une ou plusieurs substances d'intérêt cosmétique, pharmaceutique ou alimentaire.

20. Procédé selon l'une des revendications 8 à 19, caractérisé en ce qu'on encapsule dans les microcapsules une ou plusieurs substances d'intérêt cosmétique, pharmaceutique ou alimentaire, à l'état de solution, de suspension ou d'émulsion.

21. Procédé selon la revendication 20, caractérisé en ce qu'on peut réaliser une extrusion de la substance à encapsuler incorporée à l'intérieur du flux laminaire d'atélocollagène et de polyholosides destinés à constituer la paroi des microcapsules.

22. Procédé selon la revendication 20 ou 21, caractérisé en ce qu'on réalise une coextrusion de la substance active dissoute, en suspension ou sous forme émulsionnée dans une phase huileuse, à l'intérieur du flux laminaire d'atélocollagène et de polyholosides destinés à constituer la paroi des microcapsules.

23. Composition cosmétique, pharmaceutique ou alimentaire, caractérisée en ce qu'elle comprend des microcapsules à paroi mixte atélocollagène-polyholosides coagulés qui, de préférence, contiennent au moins en partie une substance présentant un intérêt cosmétique, pharmaceutique ou alimentaire, telles que définies à l'une des revendications 1 à 7 ou telles qu'obtenues par le procédé selon l'une quelconque des revendications 8 à 20.

24. Procédé de préparation d'une composition cosmétique, pharmaceutique ou alimentaire, caractérisé en ce qu'on incorpore au moins en partie des microcapsules à paroi mixte atélocollagène-polyholosides coagulés par un agent coagulant comprenant un cation bivalent, dans lesquelles on a de préférence encapsulé au moins en partie une substance présentant un intérêt cosmétique, pharmaceutique ou alimentaire.

## Claims

1. Microcapsules, characterized in that they comprise a combined atelocollagen/polyholoside wall, in particular glycosaminoglycans, coagulated by a coagulation agent comprising a divalent cation.

2. Microcapsules according to claim 1, characterized in that said divalent cation is selected from the group consisting of calcium, magnesium, manganese, barium, zinc, in particular in the form of a salt such as chloride or sulfate salt.

3. Microcapsules according to claim 1 or 2, characterized in that the proportion of polyholosides with respect to the atelocollagen may vary from 15 to 50% by weight.

4. Microcapsules according to one of claims 1 to 3, characterized in that said polyholosides are selected from glycosaminoglycans and dextran, the glycosaminoglycans are preferably selected from the structural glycosaminoglycans selected from the group consisting of chondroitine-4-sulfate, chondroitine-6-sulfate, dermatane-sulfate, heparane-sulfate, keratane-sulfate; as well as heparin and its derivatives.

5. Microcapsules according to one of claims 1 to 4, characterized in that the wall further comprises a substance able to be coagulated by the coagulation agent, in particular selected from a sodium alginate or casein.

6. Microcapsules according to one of claims 1 to 5, characterized in that one or more active principles in the state of solution, of suspension or of emulsion, in particular one or more substances of cosmetic, pharmaceutical or food interest, is present in the combined wall or within the microcapsules.

7. Microcapsules according to claim 5 or 6, characterized in that the constituents forming the wall of the microcapsules are the following:
| | |
|---|---|
| - atelocollagen | 1 to 1.5% by weight |
| - polyholosides | 0.3 to 0.5% by weight |
| - coagulatable substance | 0.2 to 0.4% by weight. |

8. Method for manufacturing microcapsules, characterized in that it comprises the following successive steps:
a) preparing a solution of atelocollagen and a solution of polyholosides separately;
b) mixing the solution of atelocollagen with the solution of polyholosides, so as to form a homogenous solution of atelocollagen and of polyholosides;
c) preparing a coagulation bath containing a coagulation agent comprising a divalent cation;
d) forming individual droplets from the homogenous solution of atelocollagen and of polyholosides which are made to drop into said coagulation bath, thereby obtaining microcapsules by coagulation of the atelocollagen and of the polyholosides under the effect of the coagulation agent; and
e) separating the microcapsules by any appropriate means, particularly by natural decantation after having possibly effected one or more washings.

9. Method according to claim 8, characterized in that the individual droplets are formed by laminar extrusion of the homogenous solution of atelocollagen and of polyholosides through an extrusion nozzle while subjecting the laminar flow to vibrations in order to dislocate the laminar flow into individual droplets.

10. Method according to claim 8 or 9, characterized in that individual droplets encapsulating a substance are formed by effecting through an extrusion nozzle a laminar co-extrusion of the homogenous solution of atelocollagen and of polyholosides and of the encapsulated substance, while subjecting the laminar flow to vibrations to dislocate the laminar flow into said individual droplets encapsulating said substance.

11. Method according to one of claims 8 to 10, characterized in that the atelocollagen-polyholoside mixture is made by introducing the solution of polyholosides into the solution of atelocollagen.

12. Method according to one of claims 8 to 11, characterized in that the polyholoside solution is prepared by placing the polyholosides, preferably obtained in the dry state, for example having been lyophilized, in solution in an aqueous solution of which the pH is adjusted so that, after mixture with the atelocollagen solution, the pH of the mixture is included between 5.5 and 10; said aqueous solution is preferably a basic buffer solution.

13. Method according to claim 12, characterized in that said buffer solution is selected from an aqueous solution of sodium hydroxide or an aqueous solution of a buffer obtained by neutralization of a weak acid with a strong base, such as for example sodium carbonate, sodium acetate or sodium citrate, sodium and potassium phosphates.

14. Method according to one of claims 11 to 13, characterized in that the concentration of polyholosides with respect to the concentration of atelocollagen is from 15 to 50% by weight.

15. Method according to one of claims 10 to 14, characterized in that the concentration of polyholosides in the polyholoside solution is from 0.5 to 4%, even better from 0.5 to 2%, and is preferably close to 1%.

16. Method according to one of claims 10 to 15, characterized in that the atelocollagen solution is an aqueous solution of atelocollagen having a concentration included between 0.5 and 2% by weight.

17. Method according to claim 16, characterized in that the atelocollagen solution is obtained by dissolution of atelocollagen fibers in a slightly acid aqueous solution, such as 0.1 M acetic acid.

18. Method according to one of claims 8 to 17, characterized in that the polyholosides are selected from the structural polyholosides selected from the group consisting of chondroitine-4-sulfate, chondroitine-6-sulfate, dermatane-sulfate, heparane-sulfate, keratane-sulfate; as well as heparin and its derivatives; or dextran.

19. Method according to one of claims 8 to 18, characterized in that one or more desired active principles in the state of solution, suspension or emulsion, in particular one or more substances of cosmetic, pharmaceutical or food interest are introduced into the aqueous solution of atelocollagen and polyholosides.

20. Method according to one of claims 8 to 19, characterized in that one or more substances of cosmetic, pharmaceutical or food interest, in the state of solution, suspension or emulsion, are encapsulated in the microcapsules.

21. Method according to claim 20, characterized in that an extrusion may be effected of the substance to be encapsulated incorporated within the laminar flow of atelocollagen and polyholosides intended to constitute the wall of the microcapsules.

22. Method according to claim 20 or 21, characterized in that a co-extrusion is effected of the active substance dissolved, in suspension or in emulsified form in an oily phase, within the laminar flow of atelocollagen and polyholosides intended to constitute the wall of the microcapsules.

23. Cosmetic, pharmaceutical or food composition, characterized in that it comprises microcapsules with combined atelocollagen-polyholoside coagulated wall which preferably contain at least in part a substance presenting a cosmetic, pharmaceutical or food interest, such as defined in one of claims 1 to 7 or such as obtained by the method according to any one of claims 8 to 20.

24. Method for preparing a cosmetic, pharmaceutical or food composition, characterized in that there are incorporated at least in part microcapsules with combined atelocollagen-polyholoside wall coagulated by a coagulation agent, comprising a divalent cation, in which a substance presenting a cosmetic, pharmaceutical or food interest has preferably been encapsulated at least in part.

## Patentansprüche

1. Mikrokapseln, dadurch gekennzeichnet, daß sie eine Wand, zusammengesetzt aus Atelocollagen und Stärkehydrolysaten, insbesondere Glucosaminoglykanen, umfassen, welche mit einem ein bivalentes Kation enthaltenden Koagulationsmittel koaguliert ist.

2. Mikrokapseln nach Anspruch 1, dadurch gekennzeichnet, daß das bivalente Kation ausgewählt ist aus der Gruppe bestehend aus Calcium, Magnesium, Mangan, Barium, Zink, insbesondere in Form eines Salzes, wie des Chlorid- oder Sulfatsalzes.

3. Mikrokapseln nach Anspruch 1 oder 2, dadurch gekennzeichnet, daß das Verhältnis von Stärkehydrolysaten zu Atelocollagen von 15 bis 50 % Masse variieren kann.

4. Mikrokapseln nach einem der Ansprüche 1 bis 3, dadurch gekennzeichnet, daß die Stärkehydrolysate ausgewählt sind aus Glucosaminoglykanen und Dextran, wobei die Glucosaminoglykane vorzugsweise ausgewählt sind aus Struktur-Glucosaminoglykanen, ausgewählt aus der Gruppe bestehend aus Chondroitin-4-sulfat, Chondroitin-6-sulfat, Dermatansulfat, Heparansulfat, Keratansulfat, sowie Heparin und Derivaten davon.

5. Mikrokapseln nach einem der Ansprüche 1 bis 4, dadurch gekennzeichnet, daß die Wand weiters eine mit dem Koagulationsmittel koagulierbare Substanz, insbesondere ausgewählt aus Natriumalginat oder Kasein, umfaßt.

6. Mikrokapseln nach einem der Ansprüche 1 bis 5, dadurch gekennzeichnet, daß ein oder mehrere Wirkstoffe im gelösten, suspendierten oder emulgierten Zustand, insbesondere eine oder mehrere Substanzen von kosmetischer, pharmazeutischer oder alimentärer Bedeutung, in der Mischwand oder im Inneren der Mikrokapseln vorhanden sind.

7. Mikrokapseln nach Anspruch 5 oder 6, dadurch gekennzeichnet, daß die Bestandteile der Wand der Mikrokapseln wie folgt sind:
| | |
|---|---|
| - Atelocollagen | 1 bis 1,5 % Masse |
| - Stärkehydrolysate | 0,3 bis 0,5 % Masse |
| - Koagulierbare Substanz | 0,2 bis 0,4 % Masse. |

8. Verfahren zur Herstellung von Mikrokapseln, dadurch gekennzeichnet, daß es die folgenden Schritte der Reihe nach umfaßt:
a) man bereitet separat eine Atelocollagenlösung und eine Lösung von Stärkehydrolysaten;
b) man mischt die Atelocollagenlösung mit der Lösung von Stärkehydrolysaten derart, daß eine homogene Lösung von Atelocollagen und Stärkehydrolysaten gebildet wird;
c) man bereitet ein Koagulierbad, enthaltend ein Koagulationsmittel mit einem bivalenten Kation;
d) man bildet einzelne Tröpfchen aus der homogenen Lösung von Atelocollagen und Stärkehydrolysaten, die man in das Koagulierbad fallen läßt, wobei man so durch Koagulation des Atelocollagens und der Stärkehydrolysate unter der Wirkung des Koagulationsmittels Mikrokapseln erhält; und
e) man trennt die Mikrokapseln mit irgendeinem geeigneten Mittel, insbesondere durch natürliches Dekantieren, nachdem gegebenenfalls eine oder mehrere Waschungen durchgeführt worden sind.

9. Verfahren nach Anspruch 8, dadurch gekennzeichnet, daß man einzelne Tröpfchen durch laminare Extrusion der homogenen Lösung von Atelocollagen und Stärkehydrolysaten durch eine Extrusionsdüse bildet, indem die laminare Strömung zwecks Zerteilens der laminaren Strömung in einzelne Tröpfchen in Vibrationen versetzt wird.

10. Verfahren nach Anspruch 8 oder 9, dadurch gekennzeichnet, daß man einzelne eine Substanz einkapselnde Tröpfchen bildet, indem durch eine Extrusionsdüse eine laminare Co-Extrusion der homogenen Lösung von Atelocollagen und Stärkehydrolysaten und der eingekapselten Substanz vorgenommen wird, wobei die laminare Strömung zwecks Zerteilens der laminaren Strömung in die die Substanz einkapselnden einzelnen Tröpfchen in Vibrationen versetzt wird.

11. Verfahren nach einem der Ansprüche 8 bis 10, dadurch gekennzeichnet, daß man die Atelocollagen-Stärkehydrolysat-Mischung herstellt, indem man die Lösung von Stärkehydrolysaten in die Lösung von Atelocollagen einbringt.

12. Verfahren nach einem der Ansprüche 8 bis 11, dadurch gekennzeichnet, daß die Lösung von Stärkehydrolysaten hergestellt wird, indem man Stärkehydrolysat, das vorzugsweise im trockenen Zustand erhalten wurde, beispielsweise durch Lyophilisieren, in einer wässerigen Lösung löst, deren pH-Wert derart geregelt wird, daß er nach dem Mischen mit der Atelocollagenlösung zwischen 5,5 und 10 liegt, wobei die wässerige Lösung vorzugsweise eine basische Pufferlösung ist.

13. Verfahren nach Anspruch 12, dadurch gekennzeichnet, daß die Pufferlösung ausgewählt ist aus einer wässerigen Sodalösung oder einer wässerigen Pufferlösung, die durch Neutralisieren einer schwachen Säure mit einer starken Base erhalten wurde, wie beispielsweise Natriumcarbonat, Natriumacetat oder Natriumcitrat, Natriumphosphat und Kaliumphosphat.

14. Verfahren nach einem der Ansprüche 11 bis 13, dadurch gekennzeichnet, daß die Konzentration an Stärkehydrolysaten in bezug auf die Konzentration an Atelocollagen 15 bis 50 % Masse beträgt.

15. Verfahren nach einem der Ansprüche 10 bis 14, dadurch gekennzeichnet, daß die Konzentration an Stärkehydrolysaten in der Stärkehydrolysatlösung 0,5 bis 4 %, noch besser 0,5 bis 2 %, vorzugsweise um 1 %, beträgt.

16. Verfahren nach einem der Ansprüche 10 bis 15, dadurch gekennzeichnet, daß die Atelocollagenlösung eine wässerige Lösung von Atelocollagen mit einer Konzentration zwischen 0,5 und 2 % Masse ist.

17. Verfahren nach Anspruch 16, dadurch gekennzeichnet, daß die Atelocollagenlösung durch Auflösen von Atelocollagenfasern in einer leicht sauren wäserigen Lösung, wie 0,1 M Essigsäure, erhalten wird.

18. Verfahren nach einem der Ansprüche 8 bis 17, dadurch gekennzeichnet, daß die Stärkehydrolysate ausgewählt sind aus Struktur-Stärkehydrolysaten, ausgewählt aus der Gruppe bestehend aus Chondroitin-4-sulfat, Chondroitin-6-sulfat, Dermatansulfat, Heparansulfat, Keratansulfat, sowie Heparin und Derivaten davon oder Dextran.

19. Verfahren nach einem der Ansprüche 8 bis 18, dadurch gekennzeichnet, daß man in die wässerige Lösung von Atelocollagen und Stärkehydrolysaten einen oder mehrere erwünschte Wirkstoffe im gelösten, suspendierten oder emulgierten Zustand, insbesondere eine oder mehrere Substanzen von kosmetischer, pharmazeutischer oder alimentärer Bedeutung, einbringt.

20. Verfahren nach einem der Ansprüche 8 bis 19, dadurch gekennzeichnet, daß man eine oder mehrere Substanzen von kosmetischer, pharmazeutischer oder alimentärer Bedeutung im gelösten, suspendierten oder emulgierten Zustand in die Mikrokapseln einkapselt.

21. Verfahren nach Anspruch 20, dadurch gekennzeichnet, daß man eine Extrusion der einzukapselnden Substanz vornehmen kann, die in der laminaren Strömung von Atelocollagen und Stärkehydrolysaten, welche die Wand der Mikrokapseln bilden sollen, enthalten ist.

22. Verfahren nach Anspruch 20 oder 21, dadurch gekennzeichnet, daß man eine Co-Extrusion der in einer öligen Phase gelösten, suspendierten oder emulgierten aktiven Substanz in der laminaren Strömung von Atelocollagen und Stärkehydrolysaten, welche die Wand der Mikrokapseln bilden sollen, vornimmt.

23. Kosmetische, pharmazeutische oder alimentäre Zusammensetzung, dadurch gekennzeichnet, daß sie Mikrokapseln mit einer koagulierten Wand, zusammengesetzt aus Atelocollagen und Stärkehydrolysaten, welche vorzugsweise zumindest teilweise eine Substanz von kosmetischer, pharmazeutischer oder alimentärer Bedeutung enthalten, nach einem der Ansprüche 1 bis 7, oder erhalten durch das Verfahren nach einem der Ansprüche 8 bis 20, umfaßt.

24. Verfahren zur Herstellung einer kosmetischen, pharmazeutischen oder alimentären Zusammensetzung, dadurch gekennzeichnet, daß man zumindest teilweise Mikrokapseln mit einer Wand, zusammengesetzt aus Atelocollagen und Stärkehydrolysaten, die mit einem ein bivalentes Kation enthaltenden Koagulationsmittel koaguliert wurden, einarbeitet, in denen vorzugsweise zumindest teilweise eine Substanz von kosmetischer, pharmazeutischer oder alimentärer Bedeutung eingearbeitet ist.
